# EUROPEAN PATENT APPLICATION

(11) **EP 4 663 116 A1**
(43) Date of publication of application: **17.12.2025**
(21) Application number: 24315293.1
(22) Date of filing: 14.06.2024
(51) Int. Cl.: A61B 5/06, A61B 5/339, A61B 5/349, A61B 5/00

(54) **ASSISTANCE DEVICE FOR IMPROVED ELECTRODE PLACEMENT**

(71) Applicant: Sorin CRM SAS, 92140 Clamart (FR)
(72) Inventor: Hernandez, Alfredo, 35510 Cesson-Sévigné (FR)
(74) Representative: Page, White & Farrer Germany LLP

(57) **Abstract**

Assistance device for improved electrode placement

This invention relates to an assistance device (e.g., user interface) and a method of assisting in placement of a pacing electrode in a target tissue area of a patient based on sensed electric signals, wherein a set of electrocardiogram (ECG) and/or electrogram (EGM) and/or accelerometer and/or bio-impedance (BioZ) signals is acquired in a concurrent manner, a real-time signal processing chain is applied to the acquired signals in order to maximize the signal to noise ratio, detect and extract individual beats (either spontaneous or stimulated), extract meaningful features from these individual beats and provide a quantitative, detailed morphological analysis, and then the evolution of these markers is displayed in real-time by means of "shadow plots" and accompanying extracted features.

## Description

### FIELD OF THE INVENTION

The invention relates to electrode placement for cardiac therapy systems for controlling optimal cardiac activation of a patient.

### BACKGROUND OF THE INVENTION

The heart is a complex organ precisely controlled by the interplay of electrical and mechanical phenomena. It consists of four chambers (left and right atria (LA, RA) and left and right ventricles (LV, RV)) connected by four valves, which act in concert to regulate its filling, ejection, and overall pump function.

Different stimulation electrode placements and different electrical activation sequences of cardiac pacing may lead to different mechanical pump efficiencies of a stimulated heart. What is needed is a fast and homogenous contraction of heart ventricles to optimize pump efficiency.

Traditional pacing sites (such as the right ventricular apex (RVA)) may provide a stable lead position with low displacement rate but are not very effective in optimizing LV contraction (representing about 80% of the heart mass). Long-term RVA pacing may have deleterious effects on the left ventricular function by inducing an iatrogenic left bundle branch block (LBBB), which can have strong influence on left ventricle hemodynamic performances. This observation led to a reassessment of traditional approaches and to a research of alternative pacing sites, in order to get to more physiological pattern of ventricular activation and to avoid deleterious effects.

Conduction system pacing (CSP) has been proposed as a technique of pacing that involves implantation of permanent pacing leads along different sites of the cardiac conduction system and includes His bundle pacing and left bundle branch pacing. Furthermore, the cardiac conduction system (CCS) consists of distinctive components that initiate and conduct an electrical impulse required for the coordinated contraction of the cardiac chambers. Lead placement for CSP can be targeted either at the bundle of His, known as His bundle pacing (HBP), or at the region of the left bundle branch (LBB), known as LBB pacing (LBBP). LBBP has emerged as an alternative method for delivering physiological pacing to achieve electrical synchrony of the LV, especially in patients with infranodal atrioventricular block and/or LBBB. The proximal left bundle branches (LBBs) run through the LV septum and fan out to form a wider target for pacing compared to the His bundle. A technique for LBBP has been developed using a ventricular transseptal approach (i.e., pacing the LV from the RV). LBBP has been reported to offer low pacing thresholds and large R waves, and because the distal conduction system is targeted, has a lower theoretical risk for development of distal conduction block.

However, challenges concerning minimization of the impact of the pacing device through the septum (e.g., arteries permanent damages), downsizing and controlling the puncturing process at the septum, and ensuring long-term reliability of the pacing device exposed to septum contraction constrains remain. A major problem of LBBP is correct/optimal placement of the stimulation lead. The electrode(s) are inserted into the septum at different levels of distality with respect to the valve plane and at different depths.

### SUMMARY OF THE INVENTION

It is an object of the present invention to provide improved assistance for placement of stimulation leads.

This object is achieved by an assistance device as claimed in claim 1, a method as claimed in claim 13, and a computer program product as claimed in claim 14.

According to a first aspect, an assistance device for assisting in placement of an electrode in a body of a patient is provided, wherein the assistance device comprises:
a signal processing apparatus configured to receive measured cardiac signals and to process the measured cardiac signals by performing cycle segmentation, feature extraction and event classification in real time; and
a user interface for displaying one or more classified events of interest of the processed cardiac signals in one or more real-time visual means, in particular shadow plots, for indicating a time-dependent change of a shape or morphology of the displayed signal during the placement of the electrode. As examples, features that can be extracted may be one or more of mean, slope amplitude, slope duration, first derivative, first peak, second peak, polarity, energy, piecewise integral etc. of determined mean coherent cycles for each measurement channel and may be combined/compared between different measurement channels (e.g., time differences between peaks, polarities, sum of the energy, algebraic sum of the integrals, subtractions, etc.) to characterize the overall activation of the heart for a given stimulation pattern.

Classified events may be events that occurred during measurements (e.g., arrhythmias, different stimulation sites and fibrillation) and that can be recognized in waveforms.

According to a second aspect, a method of assisting in placement of an electrode in a body of a patient is provided, wherein the method comprises:
receiving measured cardiac signals;
processing the measured cardiac signals by performing cycle segmentation, feature extraction and event classification in real time; and
displaying one or more classified events of interest of the processed cardiac signals in one or more real-time shadow plots during the placement of the electrode.

According to a third aspect, a computer program product is provided, which comprises code means for producing the steps of the above method of the second aspect when run on a computer device.

Accordingly, the proposed solution provides a quantitative, sensitive and robust approach to quantify morphological changes obtained from different stimulation or observations sites, overcoming the problem of manual, qualitative and stressful evaluation by the clinician. Furthermore, the limited observability problem is addressed by providing shadow plot information about the mechanical and electromechanical consequences of the stimulation on different sites. A user (e.g., physician or clinician) is thus credibly assisted in performing the technical task of electrode placement by means of a continued and/or guided human-machine interaction process.

According to a first option of any one of the first to third aspects, the user interface may comprise at least one input function for parametrizing the shadow plots, memorizing and/or pausing the shadow plots, and/or displaying, and/or setting and/or controlling markers on the shadow plots.

According to a second option which may be combined with the first option or any one of the first to third aspects, the user interface may be configured to set at least one of a predetermined number of past cycles or beats of the processed cardiac signals to be represented as shadow together with a current cycle or beat, a forgetting factor of gray levels of past cycles or beats, and specific features to be detected and displayed.

According to a third option which may be combined with the first or second option or any one of the first to third aspects, the user interface may be configured to allow recording and recalling a given cycle or beat of a respective one of the shadow plots previously watched during the lead placement process and superposing this memorized cycle or beat on the morphology of the current cycle or beat of the respective shadow plot.

According to a fourth option which may be combined with any one of the first to third options or any one of the first to third aspects, the signal processing apparatus may be configured to perform the cycle segmentation by segmenting the received cardiac signals into cardiac cycles, selecting a specific cardiac cycle as reference cycle, determining a correlation between each cardiac cycle and the reference cycle, selecting a reference cycle that correlates with most of the cardiac cycles as dominant reference cycle, and determining a mean coherent cycle by averaging delay-corrected cardiac cycles that are correlated with the dominant reference cycle.

According to a fifth option which may be combined with the fourth option, the signal processing apparatus may be configured to estimate a ratio between signal and noise levels by defining all cardiac cycles that were not excluded during the cycle segmentation as the signal and by defining a result of subtracting the determined mean coherent cycle from the signal as the noise.

According to a sixth option which may be combined with any one of the first to fifth options or any one of the first to third aspects, the signal processing apparatus may be configured to transform the received cardiac signals into combined cardiac signals by projecting data of the received cardiac signals in an orthogonal space that maximizes variance of observations, wherein each axis of the orthogonal space corresponds to another principal component derived from the received cardiac signals or to another one of the received cardiac signals. In examples, an original measurement dataset obtained from one or more sensors may be transformed into another space while preserving original information to enhance the variance of observations (e.g., by generating trajectories).

According to a seventh option which may be combined with any one of the first to sixth options or any one of the first to third aspects, the signal processing apparatus may be configured to apply a feature analysis for the feature extraction on selected principal components of the received cardiac signals and to detect the event of interest from extracted desired features. As an example, features can be extracted from obtained individual beats (cycles) by determining a mean coherent cycle. This may involve selecting those cycles that are correlated to a dominant reference cycle, shifting these cycles according to a time delay (lag) with respect to the dominant reference cycle (which may be calculated by a correlation determination process), and averaging the delay-corrected (shifted) cycles. A further option to extract features of multiple measurement channels may be to use time as a parameter and combine the measurement signals/samples together so as to yield an electrical activation pattern.

According to an eighth option which may be combined with any one of the first to seventh options or any one of the first to third aspects, the signal processing apparatus may be configured to perform the event classification by applying a learning process via machine learning by using a learning dataset that is fixed in an inference mode after a learning phase and applied in the device.

According to a ninth option which may be combined with any one of the first to eighth options or any one of the first to third aspects, the signal processing apparatus may be configured to apply dynamic time warping on the received cardiac signals of different derivation vectors or on derived principal components to extract indexes of similarity of morphology and/or trajectory between different configurations.

According to a tenth option which may be combined with any one of the first to ninth options or any one of the first to third aspects, the received cardiac signals may have been acquired from an implantable medical device (e.g., by biventricular signal acquisition) and/or from an external system (e.g., an external ECG system, an electrophysiology ward or a holter system), wherein the signal processing apparatus may be configured to record the received cardiac signals simultaneously.

According to an eleventh option which may be combined with any one of the first to tenth options or any one of the first to third aspects, the received cardiac signals may comprise one or more of a set of electrocardiograms, a set of electrograms, a set of accelerometer signals, and a set of bio-impedance signals, a set of lead impedance signals, acquired in a concurrent manner.

It is noted that the above device may be implemented based on discrete hardware circuitries with discrete hardware components, integrated chips, or arrangements of chip modules, or based on signal processing devices or chips controlled by software routines or programs stored in memories, written on a computer readable media, or downloaded from a network, such as the Internet.

It shall be understood that the assistance device of claim 1, the method of claim 13, and the computer program product of claim 14 may have similar and/or identical preferred embodiments, in particular, as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a flowchart of an exemplary procedure for placing or implanting a lead device for LBBP;
Fig. 2 shows schematically a heart with an inserted lead device for LBBP;
Fig. 3 shows schematically a diagram indicating different cardiac conducting paths and related characteristic ECG morphologies for different blocking effects;
Fig. 4 shows schematically a diagram indicating different captured structures during attempted conduction system pacing and related terminology;
Fig. 5 shows a flow diagram of a procedure for assisting in electrode placement according to various embodiments;
Fig. 6 shows a diagram with exemplary waveforms of twelve different real-time ECG signals during an electrode placement for LBBP; and
Fig. 7 shows a plurality of shadow plots with related markers on a user interface for assisting in electrode placement.

### DETAILED DESCRIPTION OF EMBODIMENTS

Various embodiments of the present invention are now described based on a cardiac resynchronization therapy (CRT) system. However, the present invention can be implemented in other systems that do not deliver a CRT.

It is noted that - throughout the present disclosure - only those structural elements and functions are shown and/or described, which are useful to understand the embodiments. Other structural elements and functions are omitted for brevity reasons. Furthermore, the structure and/or function of blocks with identical reference numbers that have been described before are not described again, unless an additional specific functionality is involved.

The CRT system can be implemented as an integrated (centralized) or distributed (decentralized) system comprising at least one controller for determining and/or tracking a heart function based on a heart sensing system which comprise a plurality of intracardiac sensing electrodes or sensors for retrieving and processing cardiac information obtained from the heart of a patient.

LBBP is a relatively novel form of CSP which involves screwing a pacing lead deep into the interventricular septum from the RV in order to capture the left bundle system. This technique has shown encouraging results from observational studies, with reported success rates of 80% to 94% and significant improvements in LV function (cf. Padala SK et al.: "Left bundle branch pacing is the best approach to physiological pacing", Heart Rhythm 02, (2020) 1(1), pp 59-67).

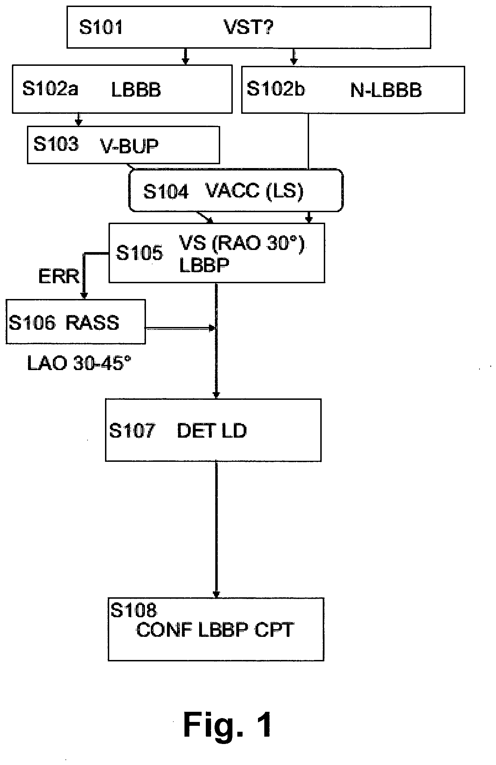

Fig. 1 shows a flowchart of an exemplary procedure for placing or implanting a lead device for LBBP.

In a first step S101 ("VST?"), the ventricular septal thickness is assessed by echo and/or scar measurements. The intraventricular septum (IVS) separates the LV and the RV and has an important role in the function of both ventricles. In an example, echocardiographic measurements may be used for measuring the septal thickness e.g. from the most distinct echoes including right and left endocardial surfaces at end diastole, which may be determined by the peak of the R wave of a simultaneously recorded ECG.

The standard surface ECG uses ten cables to obtain twelve electrical views of the heart, i.e., one cable to each limb and six cables across the chest. The different views reflect the angles at which electrodes "look" at the heart and the direction of the heart's electrical depolarization. ECG terminology has two meanings for the word "lead". First, it designates a cable used to connect an electrode to an ECG recorder. Second, it designates an electrical view of the heart obtained from any one combination of electrodes.

The chest leads V1 and V2 are placed on either side of the sternum on the fourth rib, lead V4 on the apex of the heart, lead V3 halfway between leads V2 and V4, and leads V5 and V6 horizontally laterally from lead V4. Three bipolar leads and three unipolar leads are obtained from three electrodes attached to the left arm, the right arm, and the left leg, respectively. An electrode is also attached to the right leg, but this is an earth electrode. The bipolar limb leads reflect the potential difference between two of the three limb electrodes, wherein lead I corresponds to the potential difference between right arm and left arm electrodes, lead II corresponds to the potential difference between right arm and left leg electrodes, and lead III corresponds to the potential difference between left leg and left arm electrodes. The unipolar leads reflect the potential difference between one of the three limb electrodes and an estimate of zero potential (derived from the remaining two limb electrodes). These leads are known as augmented leads. The augmented leads and their respective limb electrodes are the aVR lead at the right arm, the aVL lead at the left arm, and the aVF lead at the left leg.

Based on an intrinsic rhythm of the heart derived from ECG measurements, presence or absence of LBBB is determined in steps S102a ("LBBB") or S102b ("N-LBBB") of Fig. 1, respectively. LBBB completely modifies the electrical activation of the LV and QRS complex on the ECG. The activation of the septum, which is left-sided in physiologic conditions, originates on its right side. The electrical impulse propagates then inferiorly, to the left, and slightly anteriorly. This results in a nonhomogeneous and delayed depolarization of the LV.

The ECG criteria for an LBBB may include at least one of QRS duration greater than 120ms, absence of Q wave in leads I, V5 and V6, monomorphic R wave in leads I, V5 and V6, and ST and T wave displacement opposite to the major deflection of the QRS complex.

A simple way to diagnose an LBBB in an ECG with a widened QRS complex (> 120 ms) may be to look at lead V1. If the QRS complex is widened and downwardly deflected in lead V1, an LBBB is present. If the QRS complex is widened and upwardly deflected in lead V1, an RBBB is present.

If an LBBB has been determined in step S102a, an additional ventricular backup pacing is added in step S103 ("V-BUP").

In both cases with or without LBBB, a vein access is performed from the left side via a lead device in step S104 ("VACC (LS)").

Then, an initial site for an LBBP location at the right surface of the ventricular septum (e.g., with RAO 30°) is determined in step 5105 ("VS (LAO 30°) LBBP"). This may be achieved by placing the catheter about 1 to 1.5cm from the HBP site towards the RVA and/or by using the paced morphology, where a "W" pattern with a notch closer to nadir (deepest point of the QRS signal) in lead V1 may indicate an ideal location. The pacing lead (e.g., helical electrode) is then screwed perpendicular into the LV septum (with LAO 30-45°).

If an error ("ERR") is determined in step S105 due to a failure of fixation, a reassessment is initiated in step S106 ("RASS").

Then, in step S107 ("DET LD"), the LBBP lead depth into ventricular septum is determined. This can be achieved by at least one of observing changes in the notch in V1 lead, sheath angiography, fulcrum sign, and impedance monitoring.

The pacing lead is slowly turned into a depth of approximately 6 to 8 mm and/or based on an RBBB paced morphology while avoiding any perforation of the septum.

Finally, in step S108 ("CONF LBBP CPT"), LBB capture is confirmed based on acceptable pacing parameters. The confirmation may be based on at least one of a paced morphology of an RBBB pattern, a recording of an LBB potential, a stimulus-peak of the LVAT that shortens abruptly with increasing output or remains shortest and constant at low and high outputs, a selective LBBP and a non-selective LBBP, and a recording of a retrograde His potential or anterograde LBB potential during pacing.

In case of an RBBB, when the pacing lead is placed transseptally from the RV septum to the LV septal subendocardium in the LBB region, the paced QRS morphology of the electrocardiogram (ECG) changes from an LBBB to a right bundle branch block (RBBB) pattern, as the LV is activated earlier than the RV. However, the paced morphology could be influenced by the pacing site of the LBB, existing bundle disease, or selective or nonselective LBB capture.

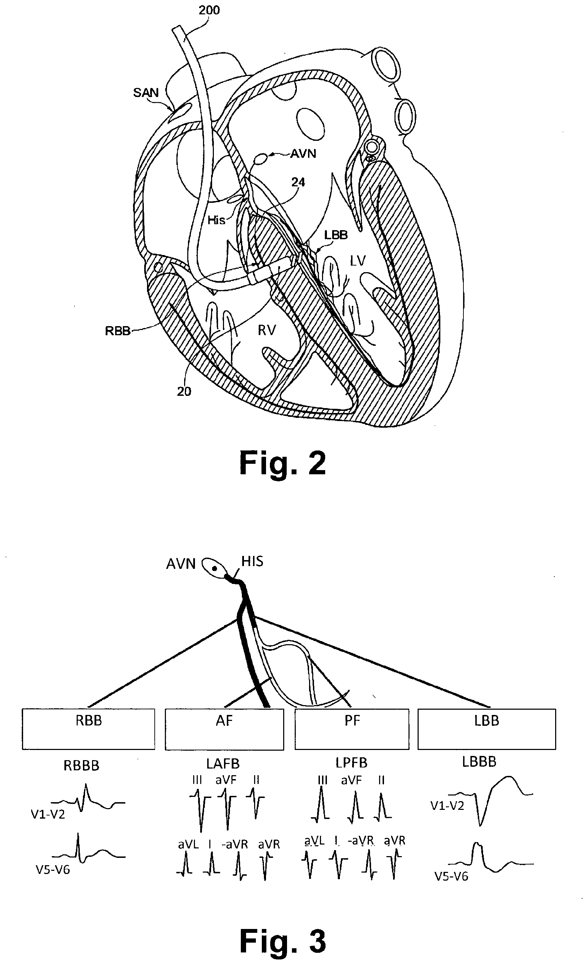

Fig. 2 shows schematically a heart with an inserted lead device 200, where the pacing lead 20 is placed for ventricular transseptal LBBP. The placement of the pacing lead 20 may be performed based on the procedure explained above in connection with Fig. 1.

In a normal cardiac function, the heartbeat starts in the heart itself due to the sinoatrial node (SAN) which is found in the top of the RA and sets the rate at which the heart contracts. It sends out electrical impulses which are carried through the muscular walls of both atria. These impulses cause atrial systole. The impulse is then passed to another node within the heart - the atrioventricular node (AVN). This node is in the lower part of the RA. Once the impulse from the SAN reaches the AVN the impulse is passed to conducting fibers which travel down the central wall of the heart. The impulse then splits and travels up the LV and RV causing them to contract simultaneously (ventricular systole).

Important elements of the conduction system of the heart are found within the septum (IVS) 24. The His bundle travels in the subendocardium, down the right side of the septum 24 for about 1cm before dividing into the LBB and RBB. The LBB continues down the right side of the septum 24, while the LBB crosses to the left side and splits into anterior and posterior divisions.

Under normal circumstances, excitation from the SAN controls the heart rhythm. An abnormality in the sinus rhythm leads to arrhythmia, which refers to abnormalities in the rate, rhythm, site of origin, and conduction of the cardiac electrical pulse. When disorders occur in specific intraventricular conduction fibers, the repolarization wave must travel through the slower muscle-muscle conduction to reach the ventricles. Classic disorders related to conditions that involve different conduction bundle branches include LBBB and RBBB.

An ECG can be used to measure and record cardiac electrical activities and thus can provide important information on cardiac functions. The ECG has been used as a standard diagnostic tool to analyze arrhythmia.

The body of the lead device 200 can be configured to improve slipperiness of a contact with a guiding catheter used for guiding the lead device (e.g., through a blood vessel) to a target area. This can be achieved by using e.g. a polyurethane (PU) material with reduced diameter to allow an advancement progress of the lead body through the guiding catheter and the lead tip through the septum 24 with limited effort.

Furthermore, the lead device 20 may comprise a fixed, non-retractable helix at its tip portion for the screwing process and may be configured to provide improved torquability, i.e., an ability to transmit safely torque to the helix (e.g., full lead body torque) and stylet-driven compatibility to ease the handling (e.g., by push transmission). In an example, a coradial lead with compatible screwing stylet (screwdriver stylet) may be provided. However, a retractable helix may as well be used in connection with the described embodiments.

A problem for the physician is to find the best location in this complex space, which derives the optimal response to stimulation, with a correct impedance level. To address this problem, the 12-leads ECG, as well as some intracardial electrogram (EGM) signals may be acquired during the lead placement procedure. The physician/clinician needs to observe these signals in real time, intra-operatively, using an electrophysiology system. While the electrode is evolving into the septal wall, different ECG and EGM morphologies can be observed and, when stimulating at these different locations, different ECG and EGM morphologies are generated according to the activation sequence, that are obtained from this stimulation site. It is thus particularly difficult to the physician/clinician to mentally perform in real-time the ECG and EGM morphology analysis process, in order to select the optimal site.

Moreover, conventionally, only electrophysiological variables have been studied. The observability of the cardiac mechanical activity has thus been neglected. Additionally, it is proposed to use other internal or external sensors, such as accelerometers or impedance sensors, to obtain a more comprehensive view of the electromechanical phenomena. The joint analysis of both electrophysiological and mechanical data is obviously far more complex that the analysis of only electrophysiological variables. A real-time automated system allowing for a quantitative, reproducible, multi-sensor analysis is thus needed.

Fig. 3 shows schematically a diagram indicating different cardiac conducting paths and related characteristic ECG morphologies for different blocking effects of the cardiac conduction system.

The upper portion of Fig. 3 shows schematically different electrical conduction paths of the heart from the AVN via the His bundle (HIS) towards the RV and LV respectively, including the right bundle branch (RBB) that activates the RV, the anterior fascicle (AF) that activates the anterior wall of the LV, the posterior fascicle (PF) that activates the posterior and inferior wall of the LV, and the left bundle branch (LBB) that provides the interventricular septum with Purkinje fibers and that is divided into the AF and PF which activate the LV.

Additionally, the different blocking effects and their characteristic morphologies or parameters in the QRS complex of measured ECG signals are indicated in the lower portion of Fig. 3.

An abnormal blocking condition of the RBB is called right bundle branch block (RBBB) and can be characterized by specific morphologies (as shown in Fig. 3) of the waveforms of ECG leads V1-V2 which show rsr', rsR', or rSR' patterns in the R- and S-waves of the QRS complex, and ECG leads V5-V6 which, together with ECG leads I and aVL, show a broad S-wave. Moreover, the QRS duration may be larger than or equal to 0.12s with normal electrical axis.

Note that the Q-wave corresponds to the first downward deflection before the R-wave of the QRS complex. The R-wave corresponds to the first upward deflection of the QRS complex and is followed by the S-wave which corresponds to the first downward deflection after the R-wave. Furthermore, a small letter of the respective Q- or R- or S-wave indicates a small wave while a capital letter of the respective Q- or R- or S-wave indicates a large wave. Moreover, one or more apostrophes called "primes" (', ", ‴ etc.) indicate subsequent waves (deflections) that may be called "double prime", "triple prime" etc.

Furthermore, an abnormal blocking condition of the AF is called anterior fascicle block (LAFB) and can be characterized by specific morphologies (as shown in Fig. 3) of the waveforms of ECG leads I-III, aVF, aVL, -aVR and aVR. ECG lead aVL shows a qR complex, ECG signals II, III and aVF show an rS complex, and ECG leads V5-V6 show a qR complex. Moreover, the QRS duration should be smaller than 0.12s with left axis deviation.

Additionally, an abnormal blocking condition of the PF is called posterior fascicle block (LPFB) and can be characterized by specific morphologies (as shown in Fig. 3) of the waveforms of ECG leads I-III, aVF, aVL, -aVR and aVR. ECG leads I and aVL show an rS complex, inferior ECG leads II, III and aVF show a qR complex, and ECG leads III and aVF always show a q-wave. Moreover, the QRS duration should be smaller than 0.12s with right axis deviation.

Further, an abnormal blocking condition of the LBB is called left bundle branch block (LBBB) and can be characterized by specific morphologies (as shown in Fig. 3) of the waveforms of ECG leads V1-V2 and V5-V6. ECG leads V1-V2 show a deep and broad S-wave or QS complex, and ECG leads V5-V6 show a broad, clumsy, positive R-wave. Moreover, the QRS duration should be larger than or equal to 0.12s and secondary ST-T changes of the QRS complex may be observed.

Thus, measured morphologies of ECG signals can be automatically compared with the above characteristic morphologies to detect blocking effects of the cardiac conduction system. This can be achieved by signal processing which may involve machine learning tools (such as neural networks), as explained further below.

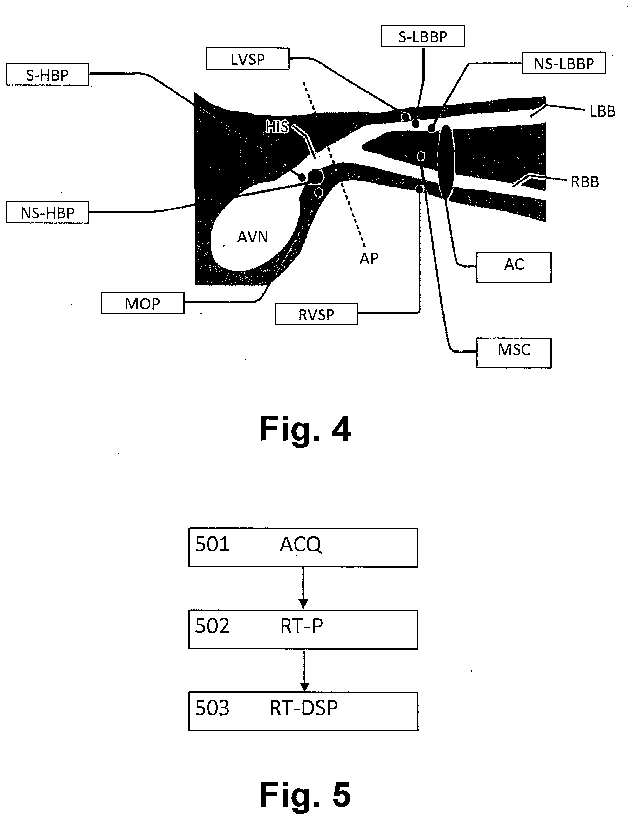

Fig. 4 shows schematically a diagram indicating different captured structures during attempted conduction system pacing and related terminology.

Similar to Fig. 3, the conduction system of the heart with AVN, HIS, LBB and RBB is shown in white color and the myocardium and membranous septum are shown in grey color. Furthermore, the annular plane (AP) is shown, which can be defined by using a combination of short and long axis reformats of the left ventricle and which can identify the insertion points of mitral valve leaflets.

The dark circular or elliptical areas represent functional virtual electrodes in different kinds of pacing sites with associated capture characteristic and morphologies indicated in the linked blocks around the schematic heart portion. The three most-left three capture morphologies correspond to His bundle pacing attempts, while the remaining capture morphologies correspond to attempts for left bundle branch area pacing.

The indicated His bundle pacing attempts include selective His bundle pacing (S-HBP) by which the His bundle can be captured alone without capture of local myocardium, non-selective His bundle pacing (NS-HBP) by which both the His bundle and the local myocardium can be captured simultaneously, and myocardium-only pacing (MOP) by which the local myocardium can be captured only without capture of the heart conduction system.

In the bundle branch area, left ventricular septal pacing (LVSP) captures the left side of the IVS without capture of the heart conduction system, selective left bundle branch pacing (S-LBBP) captures the LBB alone, without capture of the local myocardium, non-selective left bundle branch pacing (NS-LBBP) simultaneously captures the LBB and the local left ventricular septal myocardium, anodal capture (AC) can be achieved by a ring electrode that captures the right side of IVS and that may be accompanied by simultaneous LBB capture by a tip electrode, mid-septal capture (MSC) may be useful to observe the morphology during traversal of the IVS while attempting left bundle pacing for proper electrode placement, and right ventricular septal pacing (RVSP) may be useful to detect a left bundle branch block morphology at a site of approach for left bundle pacing.

Thus, the above pacing options can be selected based on a desired classification of measured signals (e.g., ECG and/or EGM and/or accelerometer and/or bio-impedance and/or lead impedance signals) based on characteristic morphologies e.g. after feature analysis, as explained below.

Fig. 5 shows a flow diagram of a procedure for assisting in electrode placement according to various embodiments.

In step 501 (ACQ), a set of ECG and/or EGM and/or accelerometer and/or bio-impedance (BioZ) and/or lead impedance signals are acquired in a concurrent manner.

The ECG signals may be acquired by the ECG leads mentioned above.

The EGM signals may be acquired from electrodes placed at one or more of a housing of an implantable medical device, the RV, the LV, the LBBP lead, coils and/or the atrium, e.g., using simultaneous acquisition.

The accelerometer may be an acceleration sensor configured to monitor heart motion in one or more spatial directions (axes). It may be attached to a cardiac wall so that one or more of cardiac movement, circumferential displacement, velocity and rotation of the heart determines the morphology of the measured acceleration signal. Events that occurred during measurements, e.g. arrhythmias, different stimulation sites and fibrillation, can be recognized in the waveforms, and may be confirmed by comparison with synchronously recorded ECG or EGM signals. The acceleration sensor may be also embedded into the can of the implant or even be applied externally, on the thorax of the patient.

Bio-Z signals may be measured by an external, non-invasive or an implantable, invasive bio-electrical sensor that can measure effectively the variations of blood content and some tissue properties through deep penetration of electrical signals into the tissue under study. To achieve this, the sensor may probe fluid inside the body by injecting a small AC current and measuring the resulting voltage through separate pairs of electrodes attached to the skin. When the Bio-Z sensor is externally placed close to the artery, it can measure arterial pulse wave from the blood volume changes. As an example, a pulse-transmit time can be measured by a small form-factor Bio-Z sensor from the delay between two voltage signals measured from two pairs of electrodes over a short distance along the artery. In addition, Bio-Z signals were validated to estimate blood pressure from wrist-worn sensors based on the pulse-transmit time and/or other features extracted from the morphology of the arterial pulse wave measured from the wrist arteries. Bio-Z sensors can be used for assessing blood flow, respiration patterns, and/or body composition. The acquired Bio-Z signal may show the arterial pulse wave with characteristic points including diastolic peak, max. slope, and systolic foot in addition to inter-beat-interval. Intra-thoracic BioZ signals may be also acquired, using a set of implanted electrodes in the heart, as well as the conductive can of the implant. A number of different dipoles may be defined by combining the different available electrodes, a couple of electrodes for current injection and another pair for voltage measurement. Intra-thoracic BioZ represents the evolution of the blood volume inside the heart as well as different mechanical modifications of the cardiac tissue properties throughout the cardiac cycle. A number of features, such as diastolic peak, max slope etc may also be estimated from the intra-thoracic BioZ.

In subsequent step 502 (RT-P), a real-time signal processing chain is applied to the acquired signals in orderto improve (e.g., maximize) the signal to noise ratio (SNR), detect and extract individual beats (either spontaneous or stimulated), extract meaningful features from these individual beats, and provide a quantitative, detailed morphological analysis which can be used to guide electrode placement e.g. by a clinician or physician.

In an example, estimation of the SNR may be calculated by defining the target signal as all cycles that were not excluded during a cycle segmentation of the acquired signals and by defining the target noise as the result of subtracting a determined mean coherent cycle (with appropriate time delay) from the signal. The SNR may then be calculated as the ratio of any statistical metric(s) of the target signal to the same metrics of the target noise (e.g., the root mean square (RMS) of the signal).

If the SNR is below a predetermined threshold for (a) given acquired signal, the measurement channel may be reconfigured (e.g., using another electrode/sensor or another combination of electrodes/sensors) before carrying out a morphological analysis as described below. Alternatively, if some/all measurement channels show a low SNR (e.g., below the predetermined threshold) for a given stimulation configuration, the parameters (e.g., pacing vector, pacing amplitudes etc.) of the stimulation may be modified. In extreme cases, when the SNR is very low for all measurement channels in multiple stimulation configurations, the integrity of the lead itself of the electrode placement may be questioned and further tests on the lead may need to be carried out.

individual beats (either spontaneous or stimulated) may be detected and extracted by cycle segmentation which may involve segmenting the (e.g., received and preprocessed) time-domain measurement signals or samples acquired in step 501 into cardiac cycles. The segmentation may be synchronized on an atrial event, or a ventricular event, or a short duration (e.g., a few milliseconds) before the atrial/ventricular event. Preferably, the same synchronization should be kept for all stimulation configurations that are considered and for all signals. During this process, outlier cycles are excluded, i.e., cycles that do not correspond to the configuration, such as premature ventricular contradictions (PVCs) in a dual-chamber pacing, sensing, acting (DDD) mode or paced ventricular cycles in atrial single chamber pacing (AAI) mode.

Meaningful features can be extracted from the obtained individual beats by determining a mean coherent cycle. This may involve selecting those cycles that are correlated to a dominant reference cycle, shifting these cycles according to a time delay (lag) with respect to the dominant reference cycle (which may be calculated by a correlation determination process), and averaging the delay-corrected (shifted) cycles.

In examples, one or more of mean, slope amplitude, slope duration, first derivative, first peak, second peak, polarity, energy, piecewise integral etc. of the determined mean coherent cycles may be extracted for each measurement channel and may be combined/compared between different measurement channels (e.g., time differences between peaks, polarities, sum of the energy, algebraic sum of the integrals, subtractions, etc.) to characterize the overall activation of the heart for a given stimulation pattern.

A further option to extract features of multiple measurement channels may be to use time as a parameter and combine the measurement signals/samples together so as to yield an electrical activation pattern. Forthis pattern to be informative, a combination process may be used to maximize complementarity between the measurement channels/samples while ignoring redundancy. Furthermore, the activation pattern may need to have a spatial significance, i.e., the measurement signals/samples may need to be combined in orthogonal planes that are independent from one other (uncorrelated) and/or independent from lead position(s) and patient anatomy.

Finally, the quantitative, detailed morphological analysis of step 502 may be achieved by extracting desired features.

To achieve this, principal components may be derived from a huge variety of measurement signals acquired from different patients (e.g., based on linear combinations) to maximize variance. This has the advantage of resulting in one representation that is independent of lead position (test performed) and patient anatomy. All other configurations from all patients can be projected in the new coordinate system and compared with one another. The spontaneous configuration as input of the principal component analyses is thus sensitive to CRT configuration changes that will be projected on the new coordinates.

Furthermore, the resulting principal components may be selected to be orthogonal to each another, so that planes of electrical activation can be computed. Since most of the information content can be described by a combination of the first and second principal components with highest variance, the first factorial plane (PC1 vs. PC2) may already provide the most significant features.

It is noted that orthogonalization concepts of embodiments described herein (such as the above-described principal component analysis based on orthogonalization) may be implemented based on other orthogonalization concepts using e.g. other kernels than maximization of variance of observations. For instance, an independent component analysis (ICA) may be applied, where statistical independence between observations may be used as orthogonalization criteria.

Once the optimal orthogonalization matrix has been obtained and the projection of the patient data has been applied to extract the principal components, further processing can be added in order to reduce the data to be processed, extract features from the subset of selected principal components, and/or classify the event in terms of feature values.

Alternatively or additionally, the desired features may be obtained from a dynamic time warping (DTW) technique which is a time series analysis for measuring similarity between two temporal sequences of acquired measurement signals of step 501, which may be compressed or delated with respect to each other.

DTW is a process that calculates an optimal match between two given sequences (e.g., time series) based on the following rules:
- Every index from the first sequence must be matched with one or more indices from the other sequence, and vice versa;
- the first index from the first sequence must be matched with the first index from the other sequence (but it does not have to be its only match);
- the last index from the first sequence must be matched with the last index from the other sequence (but it does not have to be its only match);
- the mapping of the indices from the first sequence to indices from the other sequence must be monotonically increasing, and vice versa.

An optimal match is a match that satisfies all the above rules and that has the minimal cost, where the cost may be computed as the sum of absolute differences, for each matched pair of indices, between their values.

The application of DTW on the acquired raw measurement signals of different derivation vectors or on derived principal components allows extraction of indexes of similarity of morphology and/or trajectory between different configurations. A possible example of application is the quantification of the level of morphology change between two given configurations or electrode placement sites, as well as the identification of which parts of the beat have been significantly modified.

The desired features may then be used for quantifying a level of e.g. LBB capture during electrode placement. Morphological differences on a first factorial plane (first principal component (PC1) with highest variance vs. second principal component (PC2) with second-highest variance) during the placement process and morphological modifications observed from the beginning of puncturing the septum until LBB capture.

In step 503, an evolution of markers derived from the above morphological analysis in real-time is displayed e.g. by means of "shadow plots" and the accompanying extracted features and/or by a time-series representing the evolution of the markers through time.

In each shadow plot, one or more shadow signals (shapes, morphologies) of one or more time-sequential preceding states of a target signal are displayed as shadows together with the current state of the signal. Thereby, any time-dependent change of the morphology of the displayed signal can be observed by directly comparing the current state with the shadow state, e.g., during placement of the electrode. The shadow signal is convenient because it is directly constructed from its preceding "parent" signals. The observer (e.g., clinician or physician that needs to properly place the electrode) can infer earlier morphologies (which may include an initial state as shadow signal) and the transformation of morphologies (or signal values) during the electrode placement process.

Using such a display with shadow plots for a user interface of an assistance device provides a mechanism that enables the user (e.g., clinician or physician), to make a selection or submit a command for a more precise electrode placement.

The procedure of Fig. 5 can be implemented in an assistance system or device that may comprise a memory function that allows to store previously observed morphologies of measurement signals acquired in step 501 and/or extracted desired features in a database and to compare them to a current morphology of measurement signals or extracted desired features.

Furthermore, the assistance system or device may comprise a controller or signal processor to perform the real-time processing of the above step 502 for feature extraction and morphology classification of the segmented cardiac cycles or beats in order to provide morphologic information from all the measurement channels, that can be presented in a compact diagram (e.g., shadow plots) as described in connection with step 503.

Additionally, the assistance system or device may comprise a user interface with a display as a means for assisting the user (e.g., clinician or physician) in the electrode placement process, e.g., based on a machine-learning process or machine-learning derived process that may be configured to perform the signal processing of step 502 and/or to suggest instructions to the placement procedure or automatically control a robotized placement procedure.

The machine learning process may be based on a simple neural network, e.g., a convolutional neural network (CNN, such as a shallow net). This neural network is composed of a convolution layer and a dense layer. The convolution layer basically passes its input through one or more filters (also known as kernel). A dense layer is a layer where each neuron is connected to each neuron of the preceding layer.

In an example, a database of signal morphologies e.g. of different patients may be used to train the CNN, wherein multiple morphologies obtained during advancement of an electrode through the heart tissue (e.g., septum) are applied.

Training inputs of the CNN could be for example a concatenation of principal components PC1 and PC2 e.g. derived from QRS morphologies for different placement steps for each patient.

Output targets for supervised learning of the CNN may correspond to instructions about a direction, distance and/or speed of electrode placement. The response may be defined for example as improvement, deterioration or absence of change of electrode position on a gold-standard measurement, that could be assessed with echocardiography or ECG for example.

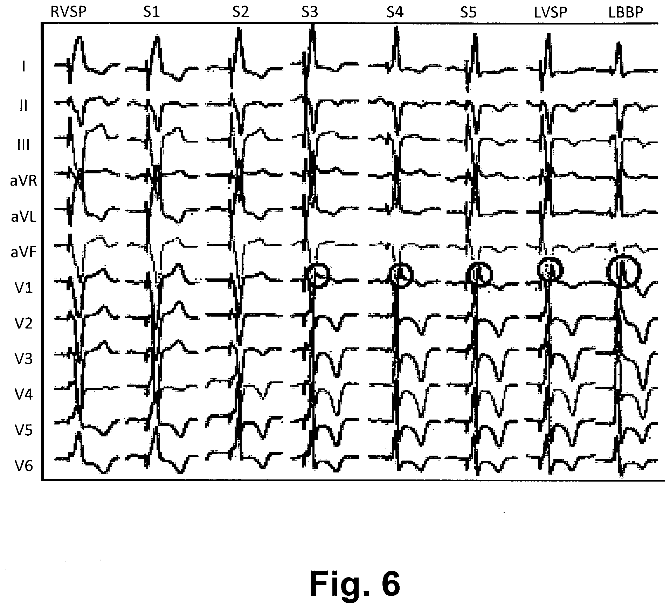

Fig. 6 shows a diagram with exemplary waveforms of twelve different real-time ECG signals as explained above during an electrode placement for LBBP. From left to right in Fig. 6, the waveforms correspond to the RVSP area of Fig. 4 at the right ventricular part of the septum, where the helix of the electrode is crewed into the septum. Then, during steps S1 to S5, the electrode is inserted through the septum by a puncturing process until it finally reaches the LVSP area of Fig. 4 and may then be retracted to the LBBP area of Fig. 4 for proper LBBP placement.

The diagram of Fig. 6 corresponds to a standard real-time monitoring representation of all available measurement data based on surface ECG signals only.

As mentioned above, embodiments could also cover acquisition, processing and display of other signals (such as EGM, BioZ, ACC etc.).

As shown in Fig. 6, the morphology of the signal acquired from ECG lead V1 includes a characteristic change (rising peak in the QRS complex) of its morphology from step S3 to final placement at the LBBP area, which is encircled in Fig. 6 for better identification. This characteristic feature of the morphology could be extracted or could be a basis for extraction in step S502 of Fig. 5 as desired feature that could be displayed in step 503 as a shadow plot of the user interface for assisting electrode placement.

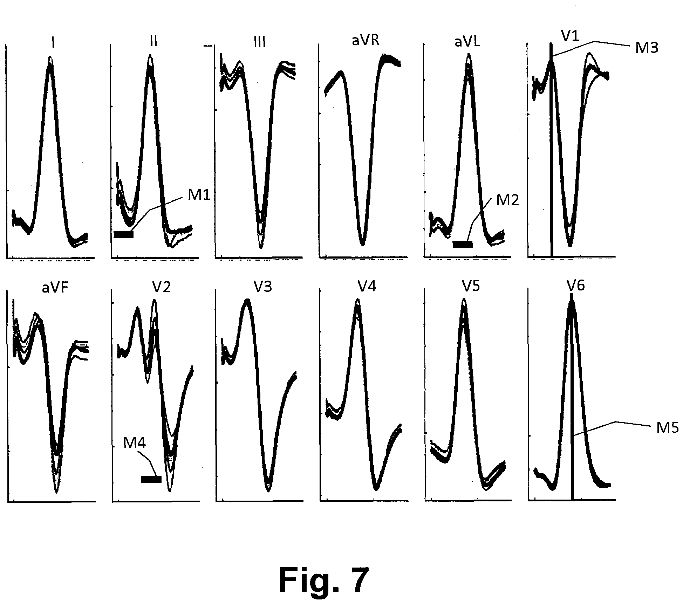

Fig. 7 shows an example of a plurality of shadow plots of ECG signals I, II, III, aVR, aVL, aVF and V1 to V6 with related markers M1 to M5 on a user interface for assisting in electrode placement.

The user interface may be a touch screen or other type of screen with mechanical input option (keyboard, buttons, mouse, etc.) on a computer-controlled assistance device, which may show the (e.g., 12) real-time ECG signals in one section and the shadow plots with related markers in another section. Additionally, control buttons or other input functions may be provided on the screen for parametrizing the shadow plots, memorizing and/or pausing the shadow plots, setting and/or controlling the markers M1 to M5 on the shadow plots.

Parametrization may comprise setting a predetermined number of past cycles or beats to be represented as shadow together with the current cycle or beat, a forgetting factor (or alpha value) of the gray levels of past cycles or beats, specific features to be detected and displayed. The memorizing input function may be configured to allow recording and recalling a given cycle or beat previously watched during a lead optimization process and superposing this memorized cycle or beat on the morphology of the current cycle.

Note again that the same kind of shadow plots can be displayed for other acquired signals (e.g., EGM, ACC, BioZ).

The markers M1, M2, M3, M4 and M5 may be used to monitor selected morphology parameters of the shadow plots, such as time periods between the stimulation instant and the main ECG waves, such as the Q-wave (M1, M3), the time period between main ECG waves, such as the period between the Q-wave and the R-Wave (M2) or the amplitudes of these significant waves, such as the amplitude of the R-wave (M5). These features may be calculated on all or a subset of the ECG leads. Additionally, derived parameters such as the time distance between the maximum peaks of the V1- and V6-signals may be displayed on the screen or represented as a time series on the user interface. A similar approach is applied to other acquired signals e.g., EGM, ACC, BioZ).

These or other individually selected markers on the shadow plots can be used together with the morphology change information derived from the shadow plots and/or the DTW results to assist the physician or clinician improve in electrode placement to thereby shorten the duration of placement and/or improve accuracy of placement.

Thus, the proposed assistance device or system credibly assists the user (e.g., physician or clinician) in performing the technical task of electrode placement by means of a continued and/or guided human-machine interaction process.

To summarize, an assistance device (e.g., user interface) and a method of assisting in placement of a pacing electrode in a target tissue area of a patient based on sensed electric signals have been described, wherein a set of electrocardiogram (ECG) and/or electrogram (EGM) and/or accelerometer and/or bio-impedance (BioZ) signals is acquired in a concurrent manner, a real-time signal processing chain is applied to the acquired signals in order to maximize the signal to noise ratio, detect and extract individual beats (either spontaneous or stimulated), extract meaningful features from these individual beats and provide a quantitative, detailed morphological analysis, and then the evolution of these markers is displayed in real-time by visual means such as "shadow plots" and accompanying extracted features, that may also be represented as time series.

While the invention has been illustrated and described in detail in the drawings and foregoing description, such illustration and description are to be considered illustrative or exemplary and not restrictive. The invention is not limited to the disclosed embodiments. The proposed assistance for electrode placement can be used for any medical devices for treatment of human beings and animals. In particular, the invention can be applied to any kind of electrodes for pacing devices or defibrillators.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. The foregoing description details certain embodiments of the invention. It will be appreciated, however, that no matter how detailed the foregoing appears in the text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

The described operations or procedures like those indicated in Fig. 5 can be implemented as program code means of a computer program and/or as dedicated hardware of the receiver devices or transceiver devices, respectively. The computer program may be stored and/or distributed on a suitable medium, such as an optical storage medium or a solid-state medium, supplied together with or as part of other hardware, but may also be distributed in other forms, such as via the Internet or other wired or wireless telecommunication systems.

## Claims

1. An assistance device for assisting in placement of an electrode in a body of a patient, the device comprising:
a signal processing apparatus (501, 502) configured to receive (501) measured cardiac signals and to process (502) the measured cardiac signals by performing cycle segmentation, feature extraction and event classification in real time; and
a user interface for displaying (503) one or more classified events of interest of the processed cardiac signals in one or more real-time visual means, in particular shadow plots, for indicating a time-dependent change of a shape or morphology of the one or more events of interest during the placement of the electrode.

2. The device of claim 1, wherein the user interface (503) comprises at least one input function for parametrizing the shadow plots, memorizing and/or pausing the shadow plots, and/or setting and/or controlling markers (M1 to M5) on the shadow plots.

3. The device of claim 1 or 2, wherein the user interface (503) is configured to set at least one of a predetermined number of past cycles or beats of the processed cardiac signals to be represented as shadow together with a current cycle or beat, a forgetting factor of gray levels of past cycles or beats, and specific features to be detected and displayed.

4. The device of any one of claims 1 to 3, wherein the user interface (503) is configured to allow recording and recalling a given cycle or beat of a respective one of the shadow plots previously watched during the lead placement process and superposing this memorized cycle or beat on the morphology of the current cycle or beat of the respective shadow plot.

5. The device of any one of the preceding claims, wherein the signal processing apparatus (501, 502) is configured to perform the cycle segmentation by segmenting the received cardiac signals into cardiac cycles, selecting a specific cardiac cycle as reference cycle, determining a correlation between each cardiac cycle and the reference cycle, selecting a reference cycle that correlates with most of the cardiac cycles as dominant reference cycle, and determining a mean coherent cycle by averaging delay-corrected cardiac cycles that are correlated with the dominant reference cycle.

6. The device of claim 5, wherein the signal processing apparatus (501, 502) is configured to estimate a ratio between signal and noise by defining all cardiac cycles that were not excluded during the cycle segmentation as the signal and by defining a result of subtracting the determined mean coherent cycle from the signal as the noise.

7. The device of any of the preceding claims, wherein the signal processing apparatus (501, 502) is configured to transform the received cardiac signals into combined cardiac signals by projecting data of the received cardiac signals in an orthogonal space that maximizes variance of observations, wherein each axis of the orthogonal space corresponds to another principal component derived from the received cardiac signals or to another one of the received cardiac signals.

8. The device of any one of the preceding claims, wherein the signal processing apparatus (501, 502) is configured to apply a feature analysis for the feature extraction on selected principal components of the received cardiac signals and to detect the event of interest from extracted desired features.

9. The device of any one of the preceding claims, wherein the signal processing apparatus (501, 502) is configured to perform the event classification by applying a learning process via machine learning by using a learning dataset that is fixed in an inference mode after a learning phase and applied in the device.

10. The device of any one of the preceding claims, wherein the signal processing apparatus (501, 502) is configured to apply dynamic time warping on the received cardiac signals of different derivation vectors or on derived principal components to extract indexes of similarity of morphology and/or trajectory between different configurations.

11. The device of any one of the preceding claims, wherein the received cardiac signals have been acquired from an implantable medical device and/or from an external system, wherein the signal processing apparatus (501, 502) is configured to record the received cardiac signals simultaneously.

12. The device of any one of the preceding claims, wherein the received cardiac signals comprise one or more of a set of electrocardiograms, a set of electrograms, a set of accelerometer signals (internal or external), a set of bio-impedance (BioZ) signals (internal or external), and a set of lead impedance signals, acquired in a concurrent manner.

13. A method of assisting in placement of an electrode in a body of a patient, the method comprising:
receiving (501) measured cardiac signals;
processing (502) the measured cardiac signals by performing cycle segmentation, feature extraction and event classification in real time; and
displaying (503) one or more classified events of interest of the processed cardiac signals in one or more real-time visual means, in particular shadow plots, for indicating a time-dependent change of a shape or morphology of the one or more events of interest during the placement of the electrode.

14. A computer program product comprising code means for generating the steps of claim 13 when run on a signal processing apparatus.
